# DEMANDE DE BREVET EUROPEEN

(11) **EP 4 566 526 A1**
(43) Date de publication de la demande: **11.06.2025**
(21) Numéro de dépôt: 24217548.7
(22) Date de dépôt: 04.12.2024
(51) Int. Cl.: A61B 5/024

(54) **ENSEMBLE OPTIQUE SUR CIRCUIT IMPRIMÉ DÉFORMABLE**

(30) Priorité: 05.12.2023 FR 2313603
(71) Demandeur: Withings, 92130 Issy-Les-Moulineaux (FR)
(72) Inventeur: Tiné, Jorys, 92130 Issy-les-Moulineaux (FR); Bedetti, Thomas, 92130 Issy-les-Moulineaux (FR)
(74) Mandataire: Withings IP

(57) **Abrégé**

Un ensemble optique comprenant :
- une unité optique (116L),
- un circuit imprimé (504) déformable, l'unité optique (116L) étant physiquement montée et électroniquement connectée au circuit imprimé (504), et
- un capteur de force (502) configuré pour déterminer une information relative à la déformation du circuit imprimé (504).

## Description

### Domaine technique

La présente description concerne un ensemble optique. Cet ensemble optique peut comprendre notamment un capteur optique.

La présente description se rapporte également aux dispositifs portables de mesures physiologiques (ci-après le dispositif de mesure), par exemple de type dispositif personnel tenable à la main (ou en anglais « *personal hand-held monitor*», PHHM) et incorporant un tel ensemble optique. De tels dispositifs sont par exemple utilisables dans le cadre d'un suivi à distance, par exemple par un médecin au cours d'une téléconsultation ou au cours d'une consultions asynchrone.

### Technique antérieure

De nombreux dispositifs pour mesurer une PhotoPléthysmoGraphie (PPG) sont connus. Les capteurs peuvent être intégrés sur une montre connectée par exemple. On peut citer notamment la Withings ScanWatch^{™}, l'Apple Watch^{™} et la Samsung Galaxy^{™}.

Il a également été proposé de combiner un capteur optique à un capteur de force, notamment pour tenter de déterminer une pression artérielle. On peut citer par exemple les documents US20180344193A1, US20170119307A1, US20220175321A1.

Néanmoins, ces dispositifs présentent un certain nombre de limitations notamment d'ergonomie et de compacité, notamment en vue d'obtenir des dispositifs facilement produits et distribués à grande échelle.

### Exposé de l'invention

La description se rapporte à des ensembles optiques permettant une mesure optique améliorée tout en offrant une architecture plus simple et compacte.

A cet effet, la présente description concerne un ensemble optique comprenant :
- une unité optique comprenant un capteur optique,
- un circuit imprimé déformable, l'unité optique étant physiquement montée et électroniquement connectée au circuit imprimé, et
- un capteur de force configuré pour déterminer une information relative à la déformation du circuit imprimé.

Dans un mode de réalisation, l'unité optique est mobile en translation selon une direction orthogonale au circuit imprimé.

Dans un mode de réalisation, l'unité optique est mobile sur une étendue comprise entre 0,01 mm et 1 mm.

Dans un mode de réalisation, l'unité optique comprend une surface d'interaction configurée pour être en contact avec un utilisateur.

Dans un mode de réalisation, l'unité optique comprend un dôme qui définit la surface d'interaction, le dôme étant monté sur le circuit imprimé de sorte que le dôme reprenne les efforts du l'utilisateur vers le circuit imprimé.

Dans un mode de réalisation, le capteur de force est un capteur de déformation piézoélectrique.

Dans un mode de réalisation, le circuit imprimé comprend une face avant et une face arrière, l'unité optique étant montée sur la face avant et le capteur de force étant monté sur la face arrière.

Dans un mode de réalisation, le capteur de force est monté sensiblement en vis-à-vis de l'unité optique.

Dans un mode de réalisation, l'ensemble comprend en outre une électrode comprenant une surface de contact configurée pour être en contact avec un utilisateur, l'unité optique étant mobile par rapport à la surface de contact.

Dans un mode de réalisation, la surface de contact de l'électrode définit une ouverture, l'unité optique étant mobile dans l'ouverture.

Dans un mode de réalisation, l'électrode est une électrode d'électrocardiogramme ECG.

Dans un mode de réalisation, l'électrode est une électrode d'impédancemétrie, IPG.

Dans un mode de réalisation, le capteur optique comprend une source de lumière et un récepteur de lumière.

Dans un mode de réalisation, au moins une partie du dôme présente une forme cylindrique ou parallélépipédique.

Dans un mode de réalisation, le bloc optique comprend un dôme définissant un volume intérieur et la surface d'interaction, le capteur optique étant arrangé dans le volume intérieur.

Dans un mode de réalisation, la surface d'interaction s'étend dans un plan parallèle à un plan tangent à la surface de contact.

Dans un mode de réalisation, la surface d'interaction est plane.

Dans un mode de réalisation, la surface d'interaction présente une dimension transversale maximale comprise entre 3 mm et 10 mm.

La présente description concerne également un dispositif comprenant un boitier et l'ensemble optique tel que défini ci-dessus, l'unité optique étant mobile par rapport au boitier entre une position de repos et une position déplacée.

Dans un mode de réalisation, au moins la surface d'interaction de l'unité optique faisant saillie hors du boitier dans la position de repos.

Dans un mode de réalisation, l'unité optique fait saillie d'une longueur comprise entre 0,5 mm et 2 mm.

Dans un mode de réalisation, l'électrode ECG est fixe par rapport au boitier.

Dans un mode de réalisation, le boitier présente une forme allongée selon une direction d'extension entre deux extrémités.

Dans un mode de réalisation, le dispositif comprend un module optique configuré pour générer les instructions permettant à la source de lumière d'émettre de la lumière et configuré pour recevoir des signaux du récepteur de lumière, notamment pour déterminer un rythme cardiaque ou une saturation en oxygène du sang de l'utilisateur.

Dans un mode de réalisation, le dispositif comprend un module de pression configuré pour calculer la pression artérielle sur la base des signaux reçus par le module PPG et le capteur de force.

Dans un mode de réalisation, le dispositif comprend une deuxième électrode ECG.

Dans un mode de réalisation, le dispositif comprend un module ECG connecté au deux électrodes ECG et configuré pour mesurer un signal ECG.

Dans un mode de réalisation, le dispositif comprend une deuxième électrode IPG.

Dans un mode de réalisation, le dispositif comprend un module IPG connecté aux deux électrodes IPG et configuré pour effectuer une mesure d'impédancemétrie.

Dans un mode de réalisation, le dispositif comprend un module d'onde configuré pour calculer une vitesse de propagation d'une onde de pouls dans un bras d'un utilisateur sur la base des signaux reçus du module ECG et du module optique.

Dans un mode de réalisation, le dispositif comprend un module de force configuré pour recevoir des signaux du capteur de force.

Dans un mode de réalisation, le dispositif est configuré pour fournir à un utilisateur une information représentative la force exercée sur l'unité optique dans la position déplacée.

Dans un mode de réalisation, le dispositif comprend un écran, l'écran étant configuré pour afficher une information représentative des signaux reçus par le module de force.

Dans un mode de réalisation, le dispositif comprend un microphone, le microphone étant configuré pour émettre une information représentative des signaux reçus par le module de force.

Dans un mode de réalisation, le dispositif comprend un vibreur, le vibreur étant configuré pour fournir par retour haptique une information représentative des signaux reçus par le module de force.

Dans un mode de réalisation, l'écran affiche un intervalle recommandé de force exercée sur l'unité optique.

Dans un mode de réalisation, le circuit imprimé déformable est fixé à chacune de ses extrémités au boitier, par exemple par une pince.

Dans un mode de réalisation, le boitier présente une forme allongée selon une direction d'extension, dans lequel le circuit imprimé est fixé au boîtier, au niveau des deux extrémités opposées le long de la direction d'extension.

### Brève description des dessins

D'autres caractéristiques, détails et avantages apparaîtront à la lecture de la description détaillée ci-après, et à l'analyse des dessins annexés, sur lesquels :
**[****Fig. 1****]** Cette figure présente deux vues en perspective, côté droit et gauche, d'un dispositif de mesure comprenant un ensemble de capteurs selon un mode de réalisation.
**[****Fig. 2****]** Cette figure présente quatre vues projetées du dispositif de la figure 1.
**[****Fig. 3****]** Cette figure présente une vue du dispositif, lors d'une manipulation en position à deux mains.
**[****Fig. 4****]** Cette figure présente trois vues (a), (b), (c) de dessus de l'ensemble de capteurs selon trois variantes différentes.
**[****Fig. 5****]** Cette figure présente une vue de dessus (a) et une coupe verticale (b) de l'ensemble de capteurs intégré dans le dispositif.
**[****Fig. 6****]** Cette figure présente deux vues en perspective (a), (b) de l'ensemble de capteurs.
**[****Fig. 7****]** Cette figure présente deux vues de côté (a), (b) de l'ensemble de capteurs en position de repos et en position déplacée.
**[****Fig. 8****]** Cette figure présente une vue en coupe verticale du dispositif, avec des composant schématisé.
**[****Fig. 9****]** Cette figure présente une vue en perspective et une vue en coupe d'une unité optique de l'ensemble de capteurs.
**[****Fig. 10****]** Cette figure présente deux vues en perspective d'une partie transparente de l'unité optique de la figure 9.
**[****Fig. 11****]** Cette figure présente deux vues projetées, face avant et arrière, d'un dispositif de mesure selon un autre mode de réalisation.
**[****Fig. 12****]** Cette figure présente une vue de dessus et une coupe verticale de l'ensemble de capteurs intégré dans le dispositif de la figure 11.
**[****Fig. 13****]** Cette figure présente une vue schématique du dispositif avec certains composants, notamment électroniques.

### Description détaillée

### Présentation générale du dispositif

La présente description va décrire plusieurs modes de réalisation et variantes d'ensembles de capteur notamment intégrés dans des dispositifs de mesures physiologiques. On parlera de « dispositif » pour simplifier le langage.

Le dispositif intègre l'ensemble de capteurs et éventuellement un ou plusieurs autres capteurs physiologiques pour mesurer des caractéristiques physiologiques (« mesure physiologique ») d'un utilisateur qui est aussi le manipulateur.

Par mesure physiologique, il est signifié des mesures de caractéristique physiologique d'un utilisateur ou utilisatrice (on parlera d'utilisateur par la suite), qui traduisent un état de santé, telles que : la température, les sons du coeur, les sons des poumons, le rythme cardiaque, l'arythmie, etc.

Le dispositif peut être portable, c'est-à-dire qu'il est léger et peu encombrant. Le dispositif peut donc être saisi aisément à une main par un utilisateur et le dispositif peut par exemple être rangé facilement dans un tiroir, un sac à main ou une poche de pantalon. A titre d'exemple, le dispositif pèse moins de 250g, voire 150g. A titre d'exemple, le dispositif présente un volume inférieur à 20x10x10 cm, voire 20x5x5cm, voire encore 15x5x5cm.

De plus, le dispositif peut être connecté, en ce sens qu'il peut envoyer des données vers un appareil tiers, tel qu'un smartphone ou un serveur. Cette connectivité permet au dispositif de fonctionner comme un dispositif de RPM, l'utilisateur devenant alors un patient d'un médecin à distance. La téléconsultation peut se faire en synchrone, avec une interaction en direct ou quasi-direct avec le médecin ou bien en asynchrone. En synchrone, le patient utilise le dispositif pour acquérir des données physiologiques qui sont immédiatement ou quasi-immédiatement transmises au médecin (quelques secondes après). En asynchrone, le patient utilise le dispositif lorsqu'il le peut et le médecin consulte les données physiologiques lorsqu'il le peut, à un moment éventuellement différent.

Le dispositif est destiné à être utilisé notamment en automesure. Il doit donc pouvoir être facilement utilisé par un utilisateur pour qu'il puisse effectuer des mesures sur lui-même.

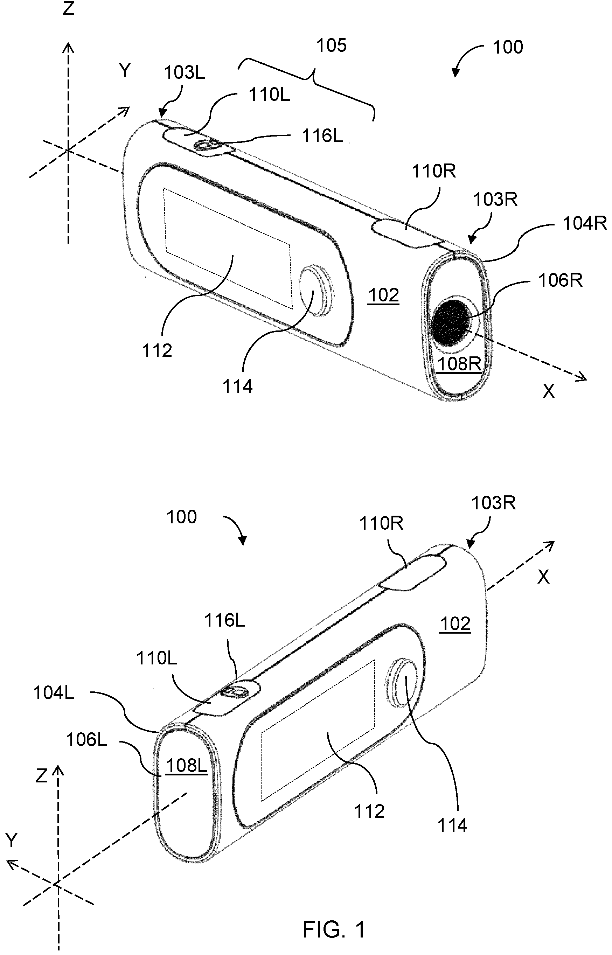

Les figures 1 à 3 représentent un dispositif 100 selon un mode de réalisation.

### Le boîtier

Le dispositif 100 comprend un boîtier 102 de forme allongée qui définit une direction d'extension X. Le boitier 102 définit également deux directions transversales orthogonales Y et Z. Par la suite, les notions de "longitudinal" et "transversal" sont définies par rapport à la direction d'extension X. Le dispositif 100 présente sa plus grande dimension selon cette direction d'extension X. La direction d'extension X est rectiligne sur les figures mais une courbure est possible dans la mesure où la manipulation du dispositif ne serait pas altérée significativement.

Le long de la direction d'extension X, le boîtier 102 comprend une première extrémité 103L (L pour "Left" ou gauche en français) et une deuxième extrémité 103R (R pour "Right" ou droit en français), opposé à la première extrémité 103L. Chaque extrémité 102L, 102R s'étend préférentiellement selon le plan transversal YZ, orthogonalement à la direction d'extension X. La première extrémité 103L définit un premier bord 104L et la deuxième extrémité 103R définit un deuxième bord 104R. Chaque bord 104L, 104R définit une courbe fermée (de forme ovoïde sur les figures du fait de la section du boîtier 102 au niveau de la première extrémité 103L et de la deuxième extrémité 103R).

Pour permettre une préhension aisée, chaque bord 104L, 104R a une longueur inférieure à 30cm, voire à 15cm.

Dans un mode de réalisation, la distance L entre les deux bords 104L, 104R, le long de la direction d'extension X est inférieur à 20cm, voire 15cm. Cette distance L permet de garantir le caractère portable du dispositif 100.

Le boîtier 102 peut avoir une forme essentiellement cylindrique, avec une section transversale dans le plan YZ (i.e. orthogonale à la direction d'extension X) de forme convexe. Dans un mode de réalisation, cette section présente deux axes de symétrie, par exemple les axes Y et Z tels que représentés sur les figures. La section est par exemple une section oblongue, comme illustré sur les figures, ou bien une section rectangulaire (avec des angles plus ou moins arrondis), ou bien une section circulaire.

Par essentiellement cylindrique, il est signifié que la section orthogonale à la direction d'extension X ne présente pas de variation de dimension significative.

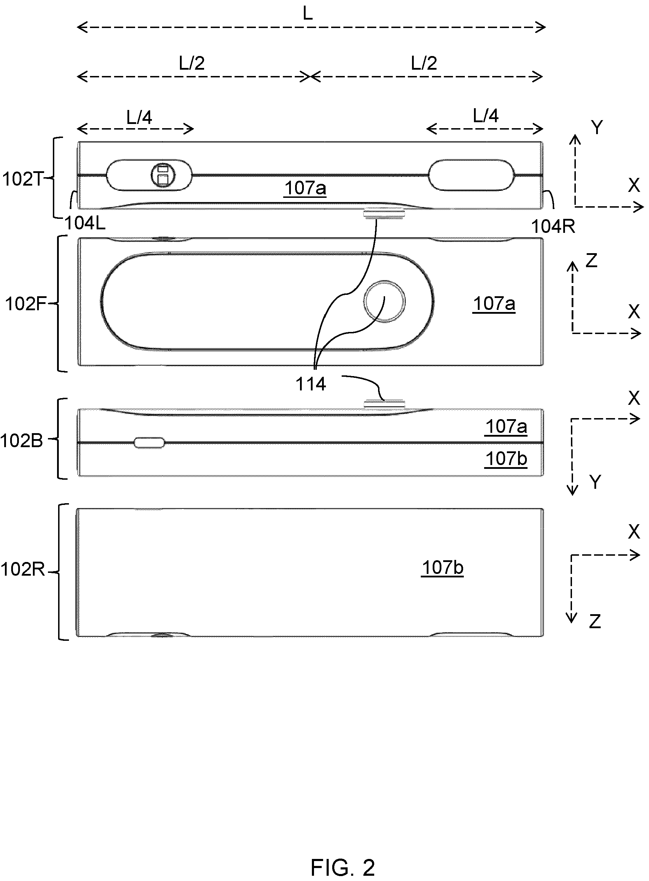

Le boîtier 102 est typiquement en matériau plastique, pour être léger, économique et isolant électriquement. Lorsque l'utilisateur tient le dispositif 100, sa ou ses mains sont majoritairement en contact avec le boîtier 102. Le boîtier 102 peut être formé de plusieurs pièces assemblées entre elle. Sur les figures 1 et 2, le boîtier 102 comprend deux coques 107, notées respectivement 107a, 107b, qui peuvent être assemblées au niveau d'une jonction parallèle à la direction d'extension X. En variante, le boitier 102 est formé d'une coque unique. D'autres types d'assemblage sont possibles.

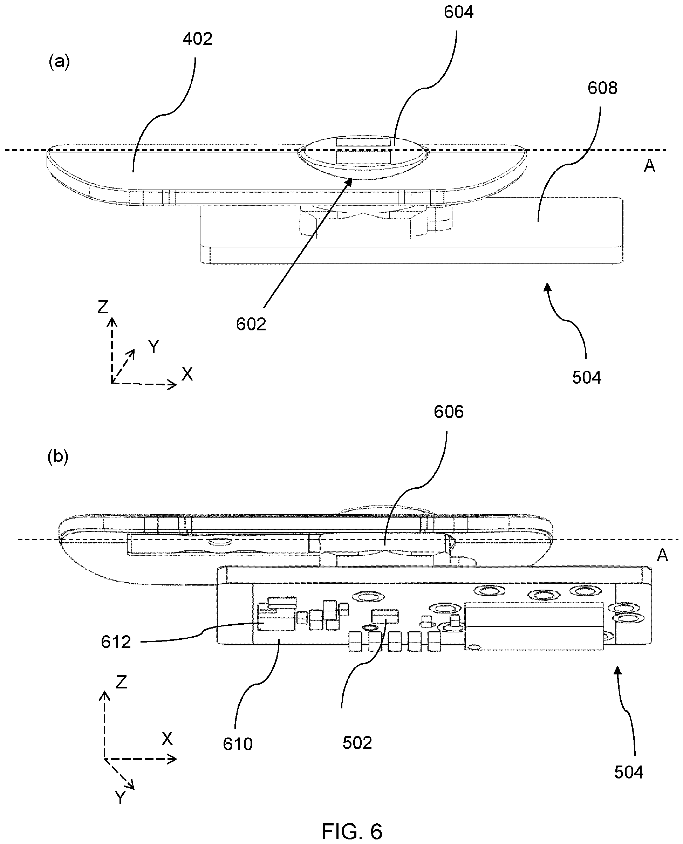

Au moins deux faces reliant les deux bords 104L, 104R peuvent être définies pour le boîtier 102. Dans le cas d'une section oblongue ou rectangulaire, on définit, comme illustré sur la figure 2, une face avant 102F (F pour "Front" ou avant en français), une face arrière 102R (R pour "Rear" ou arrière en français) (opposée à la face avant), une face supérieure 102T (T pour "Top" ou haut en français), et une face inférieure 102B (B pour "Bottom" ou bas en français), opposée à la face supérieure ; enfin les deux bords 104L, 104R définissent deux surfaces latérales. Ces noms de face sont définis par rapport à la position de manipulation à deux mains illustrées en figure 6.

Comme illustré sur la figure 2, chacune des faces supérieure 102T et inférieur 102B s'étend principalement dans un plan longitudinal XY. Chacune des faces avant 102F et arrière 102R s'étend principalement dans un plan longitudinal XZ, orthogonal au plan XY. Chacun des deux bords 104L, 104R s'étend principalement dans un plan transversal XZ, c'est-à-dire que les faces latérales sont aussi orthogonales à la direction d'extension X. Des formes arrondies pour les faces avant 102F, arrière 102R, supérieure 102T et inférieure 102U peuvent être prévues, comme illustrées sur les figures, pour ne pas avoir d'arête ou ainsi faciliter la préhension.

Les faces avant 102F et arrière 102R ont une hauteur (la dimension en Z) plus important que la profondeur (la dimension en Y) des faces inférieure 102B et supérieure 102T. Dit autrement, le boîtier 102 est plus haut que profond.

Les faces avant 102F et arrière 102R ont une longueur (la dimension en X) plus importante que la hauteur (la dimension en Z). Dit autrement, le boîtier 102 est plus long que haut.

Ces considérations de taille s'appliquent similairement au dispositif 100. Le dispositif 100 à une longueur (dimension en X) qui est supérieure à une hauteur (dimension en Z) qui est elle-même supérieure à une profondeur (dimension en Y). Par exemple, la longueur est 3 fois supérieure à la hauteur et la hauteur est 1,5 fois supérieure à la profondeur. Ces dimensions correspondent aux volumes précités pour le dispositif (sous la forme dimension en X, dimensions en Y, dimensions en Z).

Le dispositif 100 comprend un ensemble de capteurs 105 qui sera décrit plus en détail par la suite.

Le dispositif 100 comprend en outre préférentiellement un ou plusieurs capteurs physiologiques additionnels 106 disposés à différents endroits du boitier 102 et permettant d'effectuer des mesures physiologiques additionnelles qui génèrent des données physiologiques.

### L'affichage

Le dispositif 100 comprend un affichage 112, par exemple un écran (illustré en pointillé sur la figure 1 car le contour de l'écran est invisible pour l'utilisateur, ou a minima peu visible, dans ce mode de réalisation), destiné à afficher des informations et/ou des résultats de mesure pour l'utilisateur. Dans un mode de réalisation, l'affichage 112 est configuré pour afficher des informations selon une direction de lecture parallèle et/ou transversale à la direction d'extension X. Dans un mode de réalisation, le dispositif 100 comprend un gyromètre configuré pour déterminer l'orientation dans l'espace du dispositif 100 et adapter la direction de lecture en fonction de cette orientation. Ainsi, l'affichage 112 peut être transversal lorsque l'utilisateur tient le dispositif à une main et longitudinal lorsque l'utilisateur tient le dispositif à deux mains. Dans un mode de réalisation, l'affichage 112 est positionné sur la face avant 102F du boîtier 102, de sorte que l'utilisateur puisse voir facilement l'affichage 112 lorsqu'il/elle manipule le dispositif.

### L'interface physique

Le dispositif 100 comprend en outre une interface physique 114 avec l'utilisateur, qui peut prendre la forme d'un joystick (anglicisme de "bouton de navigation" en français), de flèche, etc. L'interface physique 114 est fonctionnellement reliée à l'affichage 112 et permet de naviguer dans un menu affiché sur l'affichage 112. L'interface physique 114 peut être positionné sur la face avant 102F du boîtier 102.

Pour simplifier la navigation, l'affichage 112 et l'interface physique 114 sont positionnés côte à côte, par exemple sur la face avant 102F. Dans le mode de réalisation de la figure 1, l'interface physique 114 est positionnée du côté de la deuxième extrémité 103R.

### L'ensemble de capteurs

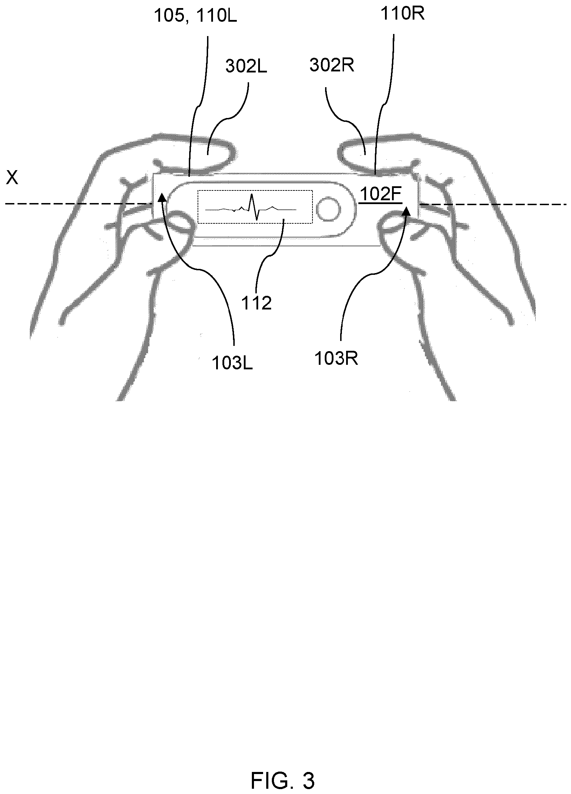

Comme indiqué précédemment, le dispositif 100 comprend au moins un ensemble de capteurs 105. L'ensemble de capteurs 105 est destiné à recevoir un doigt de l'utilisateur (index ou pouce par exemple). En particulier, comme illustré sur la figure 3, l'ensemble de capteurs 105 est positionné sous un doigt, notamment l'index 302, en position de préhension à deux mains du dispositif 100.

L'ensemble de capteurs 105 est par exemple positionné sur le boitier 102 et peut être arrangé à différents emplacements du boîtier 102. L'ensemble de capteurs 105 est notamment situé à proximité d'une extrémité, notamment de la première extrémité 103L. Selon un mode de réalisation, « à proximité d'une extrémité » signifie « entre le bord de l'extrémité et strictement la moitié de la longueur L depuis le bord ». Selon un autre mode de réalisation, « à proximité » signifie « entre le premier bord 104L et strictement un quart de la longueur L depuis le premier bord 104L ». Ces exemples sont visibles en figure 2, avec les distances U2 et L/4 représentées.

Comme illustré sur les figures 1 à 3, l'ensemble de capteurs 105 peut être positionné pour que l'index 302L, 302R de l'utilisateur se positionne naturellement dessus. A cet égard, l'ensemble de capteurs 105 peut être positionné sur la face supérieure 102T.

Selon un mode de réalisation non illustré, l'ensemble de capteurs 105 tombe sous le pouce. A cet égard, l'ensemble de capteurs 105 est positionné sur la face avant 102F.

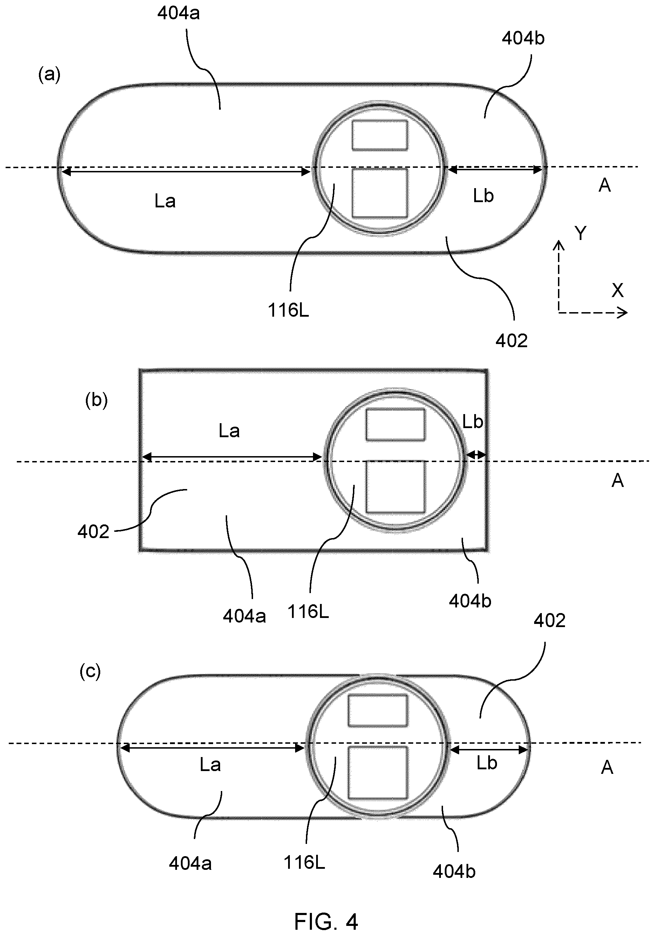

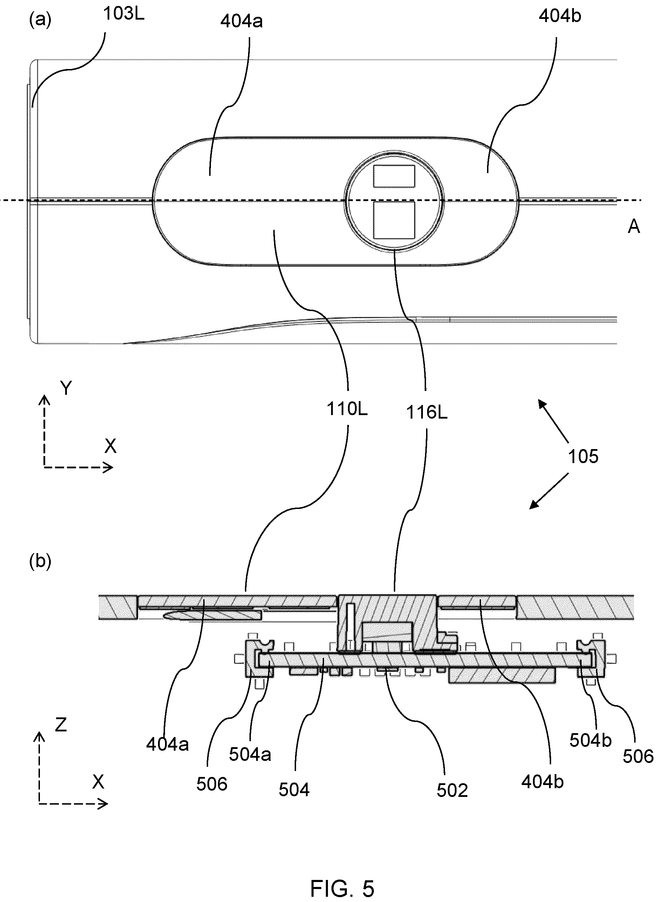

En référence aux figures 4 et 5, l'ensemble de capteurs 105 comprend une électrode 110L, aussi appelée première électrode par la suite, une unité optique 116L et un capteur de force 502.

### La première électrode

La première électrode est un capteur conducteur d'un courant électrique provenant du corps de l'utilisateur ou injecté dans le corps de l'utilisateur.

Dans un mode de réalisation, la première électrode est une électrode d'électrocardiogramme, dite première électrode ECG 110L.

En variante, la première électrode 110L est une électrode d'impédancemétrie, dite première électrode IPG 110L, notamment pour déterminer une composition corporelle de l'utilisateur (masse musculaire, masse grasse, etc).

En variante encore, la première électrode 110L est une électrode à la fois ECG et IPG. L'électrode 110L est alors reliée à une connectique permettant de passer d'une mesure à l'autre.

La première électrode 110L est préférentiellement fixe par rapport au boitier 102.

La première électrode 110L est réalisée en matériau conducteur, comme du métal (par exemple en acier inoxydable ou en alliage de titane). Dans l'exemple illustré sur les figures, la première électrode 110L se présente sous la forme d'un corps métallique. Par corps métallique, on entend une pièce dont l'épaisseur maximale est supérieure à 0,1 mm. Dans une variante, la première électrode 110L peut être formée par un revêtement conducteur déposé sur une surface (qui est elle-même conductrice ou non-conductrice).

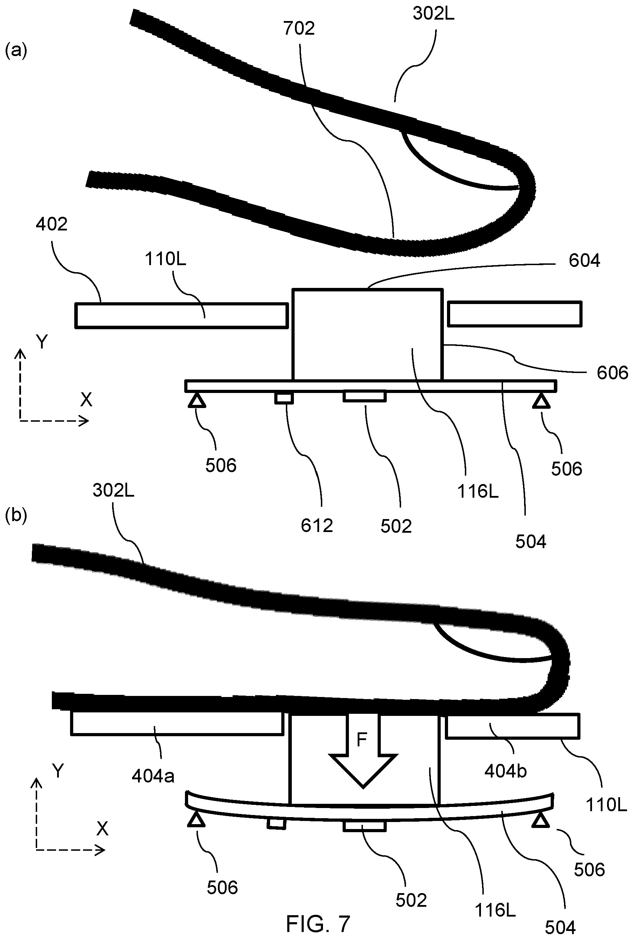

En référence aux figures 4 à 7, la première électrode 110L comprend une surface de contact 402. La surface de contact 402 est configurée pour être en contact avec un utilisateur, notamment le doigt de l'utilisateur comme illustré sur la figure 7.

Comme visible sur la figure 6, la surface de contact 402 peut présenter une forme convexe. En d'autres mots, la surface de contact 402 présente une forme bombée vers l'extérieur du boitier 102. En particulier, la surface de contact 402 s'étend dans le prolongement du boitier 102. Ainsi, il n'y a pas de discontinuité de surface entre le boitier 102 et l'électrode 110L, ce qui permet une préhension agréable pour l'utilisateur et permet un contact aisé entre l'électrode et tout partie du corps, éventuellement différente du doigt, tel que le torse par exemple.

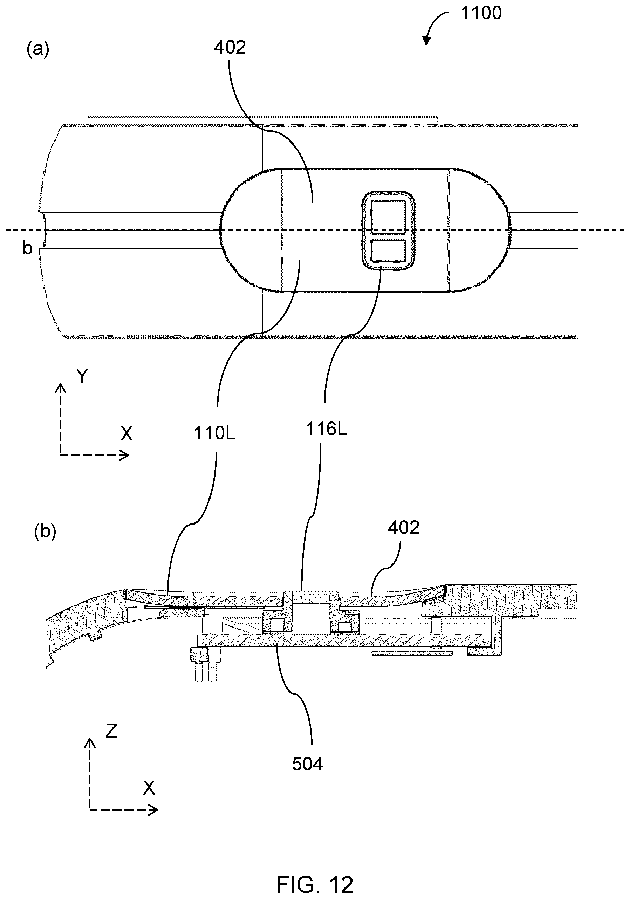

En variante, comme représenté sur la figure 12, la surface de contact 402 peut présenter une forme concave par rapport au boîtier 102. En d'autres mots, la surface de contact 402 présente une forme bombée vers l'intérieur du boitier 102 ou forme un renfoncement par rapport au boîtier. Ainsi, la surface de contact 402 forme une rigole apte à recevoir notamment un doigt pour mieux épouser sa forme et le stabiliser pendant la mesure. La surface de contact 402 guide le doigt de l'utilisateur sur l'électrode et permet une mesure stable et aisée pour l'utilisateur. Dans une variante non illustrée, la forme concave se prolonge (par exemple avec une même largeur) sur le boîtier 102 entre l'électrode 110L et le bord 104L, afin d'améliorer encore le guidage du doigt.

En variante non représentée, la surface de contact 402 présente une forme sensiblement plane.

Selon des modes de réalisations visibles sur la figure 4, la surface de contact 402 est allongée. La surface de contact 402 s'étend selon une direction principale A. La direction principale A est préférentiellement parallèle à la direction d'extension X du boitier 102. Ainsi, l'électrode 110L s'étend selon la même direction que le boitier 102. La surface de contact 402 peut présenter une forme oblongue comme représenté sur les vues (a) et (c) de la figure 4. En variante, la surface de contact 402 peut présenter une forme rectangulaire comme représenté sur la variante (b) de la figure 4. D'autres formes sont envisageables. La surface de contact 402 peut s'étendre selon une longueur comprise entre 1 cm et 5 cm selon la direction principale A. La surface de contact 402 peut s'étendre selon une largeur (la dimension selon la direction Y) comprise entre 5mm et 12mm (même lorsque la surface de contact 402 est légèrement bombée).

### L'unité optique

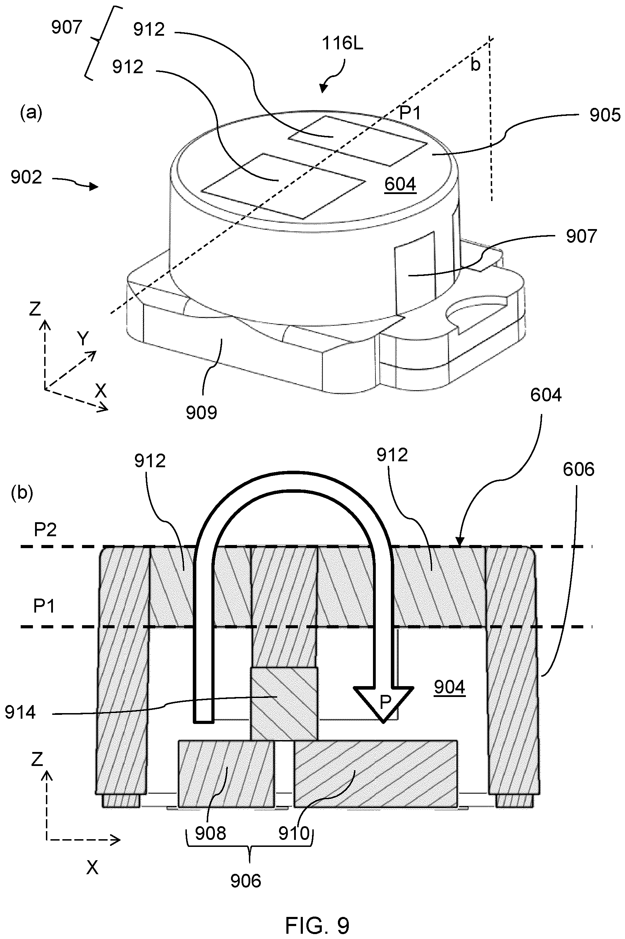

En référence à la figure 9, l'unité optique 116L comprend une surface d'interaction 604, configuré pour être en contact avec un utilisateur (par exemple l'index), et un capteur optique 906. Dans un mode de réalisation, l'unité optique 116L comprend un dôme 902 définissant un volume intérieur 904 à l'intérieur duquel est arrangé le capteur optique 906. Le dôme 902 comprend en outre la surface d'interaction 604. La structure du dôme 902 sera décrite plus en détail par la suite.

Le capteur optique 906 comprend au moins une source de lumière 908 et un récepteur de lumière 910. La source de lumière 908 est configurée pour émettre de la lumière et le récepteur de lumière 910 est configuré pour recevoir de la lumière en provenance de la source de lumière 908, notamment la lumière ayant traversé l'utilisateur, par exemple son index 302L.

Dans un mode de réalisation, la source de lumière 908 et le récepteur de lumière 910 sont alignés selon une direction orthogonale à la direction principale A selon laquelle s'étend la surface de contact 402.

Le capteur optique (notamment la source de lumière 908 et le récepteur de lumière 910) peuvent être montés sur un support 504. Le support 504 peut être un circuit imprimé (PCB, pour « *printed circuit board*» en anglais), dit circuit imprimé optique ou PCB optique par la suite. Dans ce dernier cas, le capteur optique est électroniquement connecté au PCB optique.

Le support 504 est maintenu attaché au boîtier 102. Le support 504 sera décrit plus en détail par la suite.

La source de lumière 908 peut comprendre une ou plusieurs LED, telles qu'une LED verte, une LED rouge et au moins une LED infrarouge. Le récepteur de lumière 910 peut être un photorécepteur, tel qu'une photodiode. La source de lumière 908 peut comprendre un laser.

Le dispositif 100 peut comprendre un module optique 612. Généralement, le module optique 612 comprend un convertisseur analogique-numérique (ADC) et un processeur. Le module optique 612 peut être monté sur la face arrière du circuit imprimé optique 504.Le module optique 612 est configuré pour générer les instructions permettant à la source de lumière 908 d'émettre de la lumière et configuré pour recevoir des signaux du récepteur 910. Le module optique 612 peut être configuré pour déterminer la fréquence cardiaque et/ou une saturation en oxygène du sang de l'utilisateur sur la base des signaux optiques reçus.

Le dôme 902 comprend la surface d'interaction 604 et une surface latérale 606. La surface d'interaction 604 est configurée pour être en contact avec un utilisateur, en particulier le doigt de l'utilisateur. La surface latérale 606 est configurée pour être en vis-à-vis de l'épaisseur de l'électrode 110L. La surface latérale 606 et une partie de la surface d'interaction 604 peuvent être monobloc. Le dôme 902 peut en outre comprend une base 909, aussi monobloc avec la surface latéral 606.

Comme illustré sur la figure 9, la source de lumière 908 est configurée pour émettre de la lumière hors du dôme 902 à travers la surface d'interaction 604 et le récepteur de lumière 910 est configuré pour recevoir de la lumière de l'extérieur du dôme 902 à travers la surface d'interaction 604. Ainsi, il est défini un chemin optique principal P pour la lumière entre la source de lumière 908 et le récepteur de lumière 910, passant à l'extérieur du dôme 902.

La surface d'interaction 604 est préférentiellement plane. En particulier, la surface d'interaction 604 s'étend dans un plan XY parallèle à un plan tangent à la surface de contact 402. Toutefois, la surface d'interaction 604 peut être légèrement bombée.

La surface d'interaction 604 peut présenter une dimension transversale maximale (selon la direction Z) comprise entre 3 mm et 10 mm. En particulier, la surface d'interaction 604 est préférentiellement ronde. La dimension transversale maximale est alors le diamètre.

Dans un mode de réalisation, la surface d'interaction 604 présente une aire deux fois, avantageusement trois fois, inférieure à l'aire de la surface de contact 402.

### Décentrage longitudinal de l'optique dans l'électrode

Selon des modes de réalisations représentés sur la figure 4, la surface de contact 402 comprend au moins deux sections 404 dont une première section 404a et une deuxième section 404b. Les deux sections 404a, 404b sont connectées électriquement de sorte à former l'électrode 110L. L'électrode 110L forme alors une seule et même électrode. La première section 404a et la deuxième section 404b sont disposées le long de la direction principale A. La surface d'interaction 604 de l'unité optique 116L est positionnée le long de la direction principale A entre la première section 404a et la deuxième section 404b. La première section 404a présente préférentiellement une longueur La supérieure à une longueur Lb de la deuxième section 404b le long de la direction principale A. Préférentiellement, la longueur La de la première section 404a est au moins 50% supérieure, avantageusement 100% supérieure, à la longueur Lb de la deuxième section 404b. On parle de décentrage longitudinal de l'unité optique 116L au sein de la surface de contact 402.

Comme illustré sur la figure 7, ce décentrage de l'unité optique 116L permet de maximiser le contact de l'électrode et du doigt de l'utilisateur. En effet, l'utilisateur place la pulpe de son index sur la surface d'interaction 604 de l'unité optique 116L. La partie proximale de l'index se place sur la première section 404a et la partie distale de l'index, plus petite que la partie proximale, se place sur la deuxième section 404b. Ainsi, l'ensemble, ou a minima la majorité, de l'électrode est recouverte, ce qui permet un meilleur signal électrique, notamment ECG, et limite les perturbations extérieures.

Comme représenté sur la vue (c) de la Figure 4, la première section 404a et la deuxième section 404b de la surface de contact 402 peuvent être disjointes (par exemple visuellement séparées). La première section 404a et la deuxième section 404b peuvent être deux pièces différentes, tout en étant connectées électriquement, par exemple par un fil électrique s'étendant entre les deux sections 404 à l'intérieur du boitier 102, ou bien une même pièce dont la surface de contact comprend les deux sections 404a, 404b disjointes.

Néanmoins, pour que le doigt soit à la fois en contact avec la surface d'interaction 604 de l'unité optique 116L et la surface de contact 402 de l'électrode, la surface d'interaction 604 de l'unité optique 116L a une dimension selon la direction Y inférieure ou égale à celle de l'électrode 110L (c'est-à-dire la largeur de l'électrode).

En variante, comme représenté sur les vues (a) et (b) de la Figure 4, la première section 404a et la deuxième section 404b peuvent être jointes. Autrement dit, les deux sections 404 forment une unique pièce.

Dans cette variante, la surface de contact 402 entoure préférentiellement la surface d'interaction de l'unité optique 116L. La surface de contact 402 définit alors une ouverture 602. L'ouverture 602 est longitudinalement décentrée par rapport à la surface de contact 402 le long de la direction principale A. Par décentré, il est entendu que le barycentre de la surface de contact 402 et le barycentre de l'ouverture 602 ne sont pas confondus. L'unité optique 116L, dont la surface d'interaction 604, est arrangée dans l'ouverture 602. Dans un mode de réalisation, le centre de la surface d'interaction 604 est situé entre 55 % et 95%, préférentiellement entre 65 % et 85 % de la longueur de la surface de contact 402 selon la direction principale A.

L'électrode 110L s'étend autour de l'ouverture 602 au moins sur 1mm, notamment au niveau d'une direction transversale à la direction principale A (direction Y). Pour des raisons d'ergonomie et contact avec l'électrode, l'ouverture 602 est transversalement centrée (même distance d'électrode de part et d'autre le long de la direction Y).

Comme visible sur la figure 5, la surface de contact 402 est située à proximité d'une extrémité 103L du dispositif 100 le long de la direction d'extension X. La première section 404a étant située entre ladite extrémité 103L et la deuxième section 404b le long de la direction d'extension X. Autrement dit, dans la variante où les deux sections 404 sont jointes, l'ouverture 603 est arrangée dans la surface de contact 402 du côté opposé à ladite extrémité 103L.

Comme visible sur la Figure 3, lors de l'utilisation du dispositif 100 par l'utilisateur, les phalanges proximales des index 302L, 302R se retrouvent dans le prolongement de la direction d'extension X, tandis que la face arrière 102R repose sur les majeurs ou annulaires et que les pouces viennent s'appuyer sur la face avant 102F. Ainsi, lors de cette manipulation du dispositif à deux mains, l'index 302L se place naturellement sur l'ensemble de capteurs 105, comme visible sur la Figure 7. En particulier, la pulpe 702 de l'index 302L se place naturellement sur l'unité optique 116L de sorte à permettre une mesure optique de bonne qualité, tout en garantissant qu'une partie importante de la surface de contact 402 de l'électrode 110L soit en contact avec le doigt.

### Unité optique mobile adjacente à l'électrode

Comme visible sur les figures, l'unité optique 116L est adjacente à la surface de contact 402 de l'électrode 110L. Plus spécifiquement, la surface d'interaction 604 de l'unité optique 116L est adjacent à la surface de contact 402 de l'électrode 110L. Par adjacent, il est signifié qu'aucune pièce mécanique n'est disposée entre la surface de contact 402 et l'unité optique 116L (dans un plan au niveau de la surface de contact 402). La surface de contact 402 peut être séparée de l'unité optique 116L par un espace inférieur à 1 mm, notamment moins de 0,1 mm. En variante, la surface de contact 402 est en contact direct avec l'unité optique 116L.

Cet arrangement permet d'avoir un capteur très compact, qui interagit avec une même zone du doigt et maximise le contact entre le capteur et le doigt.

### Structure du dôme

En référence à la figure 9, au moins une partie du dôme 902 présente une forme tronconique, par exemple cylindrique. Sa base est circulaire sur les figures. La surface d'interaction 604 est alors un disque et la surface latérale 606 un cylindre. En variante, la base peut être carré ou rectangulaire de sorte à former un parallélépipède.

Le dôme 902 peut comprendre un matériau plastique. En particulier, le dôme peut être constitué entièrement de matériau plastique. En variante, le dôme peut comprendre du métal et/ou du verre.

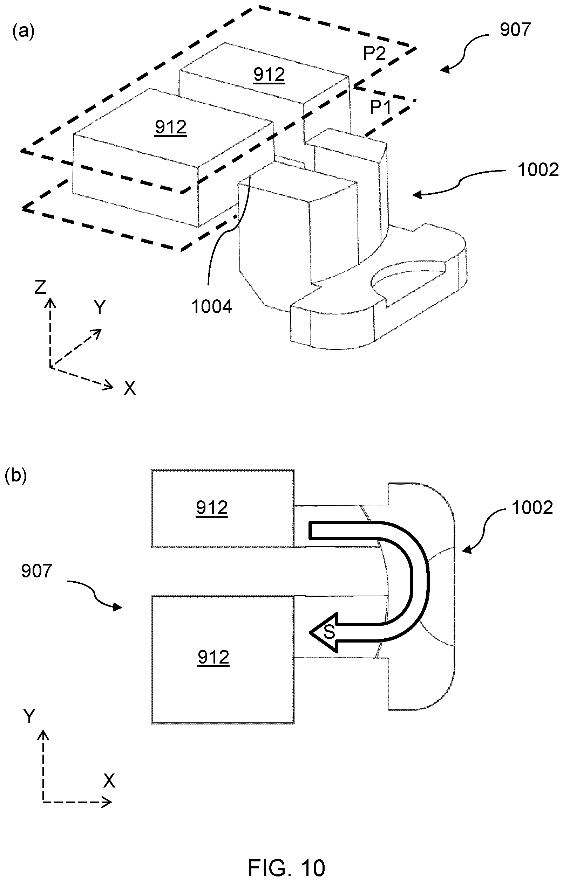

Dans un mode de réalisation, le dôme 902 comprend une pièce opaque 905 et une pièce transparente 907. La pièce transparente 907 est représentée de manière isolée sur la figure 10. La pièce transparente 907 est configurée pour laisser au moins partiellement la lumière la traverser. Au contraire, la pièce opaque 905 est configurée pour bloquer le passage de la lumière.

La pièce opaque 905 peut former la base 909, la surface latérale 606 et une partie de la surface d'interaction 604. Le dôme 902, et en particulier la base 909, peut être monté sur le support 504. Ainsi, la pièce opaque 905 transmet directement la force exercée par le doigt sur la surface d'interaction 604 au support 504.

Chaque pièce 905, 907 est monobloc. Autrement dit, chaque pièce 905, 907 est formée d'un seul bloc de matière. Chaque pièce est notamment formée d'un seul tenant de matière plastique. Les deux pièces 905, 907 sont moulées ensemble, notamment par bi-injection. La bi-injection permet la production du dôme 902 avec deux injections de matière plastique successives. En particulier, le procédé de production du dôme 902 comprend l'injection d'une matière plastique transparente dans un premier moule pour former la pièce transparente 907. Puis la pièce transparente est placée dans un deuxième moule et une matière plastique opaque est injectée dans le deuxième moule autour de la pièce transparente pour former la pièce opaque. En variante, le procédé est réalisé avec un seul moule. La pièce transparente reste alors dans le moule et la deuxième injection est faite dans ce moule. La matière opaque vient surmouler la pièce transparente pour former le dôme 902.

Le dôme 902 comprend au niveau de la surface d'interaction 604 une partie transparente 912 respective conçu pour être disposée en regard de chaque source de lumière 908 et de chaque récepteur de lumière 910 : sur les figures, il y a ainsi deux parties transparentes 912. Ainsi, les deux parties transparentes 912 sont alignées selon une direction orthogonale à la direction selon laquelle s'étend la surface de contact 402. Comme visible sur les figures, la surface d'interaction 604 est formée par une partie de la pièce opaque 905 et la pluralité de parties transparentes 912.

Comme illustré sur la figure 9, la source de lumière 908 est configurée pour émettre de la lumière hors du dôme 902 à travers la partie transparente 912 associée et le récepteur de lumière 910 est configuré pour recevoir de la lumière de l'extérieur du dôme 902 à travers la partie transparente 912 associée.

Comme visible sur la figure 10, les parties transparentes 912 sont reliées entre elle par au moins une pièce de liaison 1002. La pièce transparente 907 comprend donc la pluralité de parties transparentes 912 et l'au moins une pièce de liaison 1002. Dans un mode de réalisation, la pièce transparente 907 comprend une pluralité de parties transparentes 912 et une pièce de liaison 1002 reliant la pluralité de parties transparentes 912.

Comme visible sur la figure 9, les parties transparentes 912 sont préférentiellement à distance du capteur optique 906. Autrement dit, le capteur optique 906 n'est pas en contact avec les parties transparentes 912.

Comme visible sur les figures 9 et 10, l'épaisseur des parties transparentes 912 selon l'axe Z définissent un plan inférieur P1 et un plan supérieur P2. Chaque partie transparente 912 présente préférentiellement une forme parallélépipédique. En variante, chaque partie transparente 912 peut présenter une forme cylindrique.

En référence à la vue (b) de la figure 10, la pièce de liaison 1002 s'étend entre les deux parties transparentes 912 et forme une partie d'un chemin optique secondaire S, appelé labyrinthe optique, entre la source de lumière 908 et le récepteur 910. Le labyrinthe optique S est différent du chemin optique principal P.

La pièce de liaison 1002 est conçue de sorte que le flux lumineux passant dans le labyrinthe optique S est très inférieur, notamment au moins dix fois inférieur, au flux lumineux passant dans le chemin optique principal P. Ainsi, le flux lumineux passant dans le labyrinthe optique S peut être considéré comme négligeable par rapport au flux lumineux passant dans le chemin optique principal P. Par conséquent, le flux lumineux capté par le récepteur de lumière 910 peut être considéré comme étant essentiellement le flux provenant du chemin optique principal P ayant traversé le corps de l'utilisateur. Ainsi, le labyrinthe optique S ne perturbe pas les mesures physiologiques faites avec le capteur optique 906.

A cet effet, comme visible sur la vue (a) de la figure 10, la pièce de liaison 1002 s'étend sur au moins une portion entièrement hors de la zone délimitée par le plan inférieur P1 et le plan supérieur P2 (i.e. la zone qui est entre le plan inférieur P1 et le plan supérieur P2) de sorte que le labyrinthe S s'étende, sur une portion, intégralement hors de cette zone. Autrement dit, au moins une partie de la pièce de liaison 1002 n'est pas située entre le plan inférieur P1 et le plan supérieur P2. En particulier, la pièce transparente 907 s'étend au moins en partie selon une direction non parallèle à la surface d'interaction 604. Ainsi, la lumière parcourant le chemin optique secondaire S rencontre plusieurs changements de direction, ce qui limite grandement le flux traversant le chemin optique secondaire S. La pièce transparente 907 comprend préférentiellement des variations de sections transversales selon la direction de circulation générale de la lumière, de sorte à limiter davantage le flux lumineux dans le chemin optique secondaire S. En particulier, la transition 1004 entre chaque partie transparente 912 et la pièce de liaison 1002 forme un rétrécissement de section important (entre la section de la partie transparente 912 et la section de la pièce de liaison 1002 donc). La pièce transparente 907 peut en outre déboucher sur la surface latérale 606.

La pièce transparente 907 permet donc une production du dôme aisée tout en limitant très fortement le bypass du chemin optique secondaire S. En effet, le fait de produire le dôme 902 avec une partie transparente 907 unique, et non avec deux pièces différentes en face respectivement de la source et du récepteur, permet d'utiliser un procédé de fabrication plus rapide et simple. En particulier, avec deux pièces transparentes distinctes, il serait compliqué de former le dôme par bi-injection.

L'unité optique 116L comprend en outre un cache 914 disposé dans le volume intérieur 904. Le cache 914 est disposé entre la source de lumière 908 et le récepteur de lumière 910, de sorte que la lumière ne puisse pas circuler directement de la source de lumière 908 vers le récepteur de lumière 910 dans volume intérieur 904. Le cache 914 cloisonne le volume intérieur 904 en deux espaces distincts. La source de lumière 908 et le récepteur 910 sont disposés chacun dans un espace respectif.

Le cache 914 peut être fait d'un matériau compressible. Par exemple, le cache 914 est en élastomère. Le cache 914 peut être écrasé au montage entre le dôme 902 et le capteur optique 906 afin d'assurer qu'aucun jeu n'existe entre les deux espaces.

### Unité optique mobile

En référence à la figure 7, l'unité optique 116L est préférentiellement mobile par rapport à la surface de contact 402 de l'électrode 110L. En particulier, l'unité optique 116L est mobile en translation selon une direction orthogonale à la surface d'interaction 604 (la direction Z sur les figures, étant donné que la surface d'interaction 604 s'étend essentiellement selon les directions X et Z). L'unité optique 116L peut être mobile sur une étendue comprise entre 0,01 et 1 mm.

L'unité optique 116L est mobile entre une position de repos, représentée sur la vue (a) de la figure 7, et une position déplacée, représentée sur la vue (a) de la figure 7. Et inversement, l'unité optique 116L est mobile entre la position déplacée et la position de repos. La position de repos est la position de l'unité optique 116L lorsque l'utilisateur n'exerce aucune force sur l'unité optique 116L, notamment quand l'utilisateur n'est pas en contact avec l'unité optique 116L. La position déplacée est la position de l'unité optique 116L lorsque l'utilisateur exerce une force F sur l'unité optique 116L.

Comme visible sur les figures 6 et 7, au moins une partie de l'unité optique 116L fait saillie hors de la surface de contact 402 dans la position de repos. En particulier, la surface d'interaction 604 fait saillie hors de la surface de contact 402 dans la position de repos. Ainsi, au moins une partie de l'unité optique 116L s'étend au-delà du prolongement de la surface de contact 402. Dans la position de repos, l'utilisateur peut donc ressentir l'aspérité crée par cette saillie sous son doigt. L'unité optique 116L peut faire saille d'une longueur comprise entre 0,1 mm et 2 mm.

Il est rappelé ici que l'unité optique 116L est adjacente à la surface de contact 402 de l'électrode 110L. Plus spécifiquement, la surface d'interaction 604 de l'unité optique 116L est adjacente à la surface de contact 402 de l'électrode 110L.

### Le support

Comme visible sur les figures 5 et 6, le dôme 902 est monté sur le support 504, de sorte à fermer le volume intérieur 904 du dôme 902. Ainsi, le dôme 902, qui comprend la surface d'interaction 604 sur laquelle appuie l'utilisateur, transmet directement la force du doigt vers le support 504.

Le support 504 comprend une face avant 608 et une face arrière 610. L'unité optique 116L est montée sur la face avant 608 du support 504.

Comme expliqué ci-dessus, le support 504 peut être un circuit imprimé, dit circuit imprimé optique, sur laquelle est montée l'unité optique 116L. Le circuit imprimé optique 504 peut être fait d'une couche métallique de traces, par exemple en cuivre, liée à une couche diélectrique, par exemple en polyimide. Le dispositif 100 peut comprendre un circuit imprimé principal 802. Notamment l'affichage 112 et l'interface physique 114 sont reliés et commandé par le circuit imprimé principal 802. Le circuit imprimé optique 504 est reliée électriquement au circuit imprimé principal 802.

Dans un mode de réalisation et en référence à la figure 7, le support 504 est déformable. En particulier, le support 504 est déformable par la force d'un doigt d'un utilisateur exercé sur l'unité optique 116L lorsqu'il est mobile par rapport à l'électrode 110L. Plus précisément, le support 504 est déformable par l'unité optique 116L (notamment via le dôme 902), qui reçoit elle-même la force du doigt de l'utilisateur.

Comme mentionné précédemment, le support 504 est attaché au boîtier 102. Afin d'être déformé, dans un mode de réalisation, le support 504 est fixé au boîtier 201, notamment au niveau de deux extrémités opposées 504a, 504b (par exemple opposé le long de la direction d'extension X, comme illustré sur les figures, dans la mesure où le support 504 présente une forme allongée de le long de cette direction d'extension X). Comme visible sur la vue (b) de la figure 5, le support 504 est notamment fixé à chacune de ses extrémités, par exemple par une pince 506, au boitier 102. Ces deux pinces 506 rendent les extrémités 504a, 504b du support 504 fixe dans le référentiel du boitier 102 et ainsi le support 504 se déforme entre les deux extrémités fixes, comme représenté sur la vue (b) de la figure 7. Les pinces 506 permettent un maintien du support 504 dans la direction de déplacement de l'unité optique tout en permettant un assemblage simplifié du support dans le boitier.

### Le capteur de force

Comme visible sur le mode de réalisation des figures 6 et 7, le capteur de force 502 est un capteur de déformation du support 504. A cet égard, le capteur de déformation peut être monté sur le support 504, par exemple sur la face arrière 610 du support 504. En particulier, le capteur de force 502 est monté sur la face opposé à l'unité optique 116L. Le capteur de force 502 est monté préférentiellement sensiblement en vis-à-vis de l'unité optique 116L. Toutefois, en variante, le capteur de force 502 peut être déporté sur le support par rapport à l'unité optique 116L.

Le capteur de force 502 est préférentiellement un capteur de déformation du support 504 déformable. Le capteur de force 502 est par exemple un capteur de déformation piézoélectrique. Le capteur de force 502 est configuré pour mesurer la déformation du support 504. En particulier, le capteur de force 502 est configuré pour produire un signal permettant de déterminer une information relative à la force exercée sur l'unité optique 116L dans la position déplacée.

A cet effet, le dispositif 100 comprend un module de force 806 configuré pour recevoir des signaux du capteur de force 502 et pour en déduire une information relative à la force exercée sur l'unité optique 116L. Généralement, le module de force 806 comprend un convertisseur analogique-numérique (ADC) et un processeur. Le module de force 806 peut être monté sur le circuit imprimé principal 802 ou sur le circuit imprimé optique 504.

L'intégration du capteur de force 502 directement sur le circuit imprimé optique 504 déformable permet d'obtenir une architecture mécanique et électronique simple et compact. En effet, le circuit imprimé optique 504 sert à la fois de support de l'unité optique, de jauge de déformation en plus du support des circuits imprimés, ce qui limite les problématiques d'encombrement, de complexité, d'assemblage et de coût. Cette intégration permet en outre de s'affranchir des médiums de propagation de force et de minimiser le nombre de pièces, ce qui améliore la mesure de la force exercée par le doigt.

Dans un mode de réalisation, le dispositif 100 comprend en outre un module de pression 808 configuré pour recevoir des données issues du module optique 612 et du module de force 806 et d'en déduire une pression artérielle de l'utilisateur, notamment sur la base de l'analyse de la pulsatilité du doigt qui appui sur l'unité optique avec une pression déterminée par le module de force. Généralement, le module de pression 808 comprend un convertisseur analogique-numérique (ADC) et un processeur. Le module de pression 808 peut être monté sur le circuit imprimé principal 802 ou sur le circuit imprimé optique 504.

### Retour utilisateur

Dans un mode de réalisation, le dispositif 100 est configuré pour fournir à l'utilisateur une information représentative des signaux reçus par le module de force 806.

En particulier, l'affichage 112 peut être configuré pour afficher une information représentative des signaux reçus par le module de force 806. L'écran affiche par exemple un intervalle recommandé de force à exercer par l'utilisateur sur l'unité optique 116L, notamment pour améliorer la qualité des mesures optiques.

En variante ou en complément, le dispositif 100 peut comprendre un microphone. Le microphone est alors configuré pour émettre une information représentative des signaux reçus par le module de force 806.

En variante ou en complément, le dispositif peut comprendre un vibreur. Le vibreur est alors configuré pour fournir par retour haptique une information représentative des signaux reçus par le module de force 806.

### La deuxième électrode

Dans un mode de réalisation non représenté, le dispositif 100 comprend un deuxième ensemble de capteurs afin qu'en position de manipulation à deux mains, représentée sur la figure 3, les deux ensembles de capteurs soient positionnés sous un doigt de l'utilisateur. Le deuxième ensemble de capteurs peut être semblable à l'ensemble de capteurs 105 décrit ci-dessus. En variante, le deuxième ensemble de capteurs peut être différent et par exemple comprendre un unique capteur.

En variante représentée sur les figures, le dispositif comprend une deuxième électrode 110R positionnée à proximité de la deuxième extrémité 103R afin qu'en position de manipulation à deux mains, représentée sur la figure 3, chaque électrode 110L, 110R soit positionnée sous un doigt de l'utilisateur.

La structure de la deuxième électrode 110R est similaire à la première électrode 110L et ne sera pas décrite à nouveau. La seconde électrode 110L peut toutefois différer de la première électrode 110L notamment du fait d'une absence d'ouverture pour une unité optique.

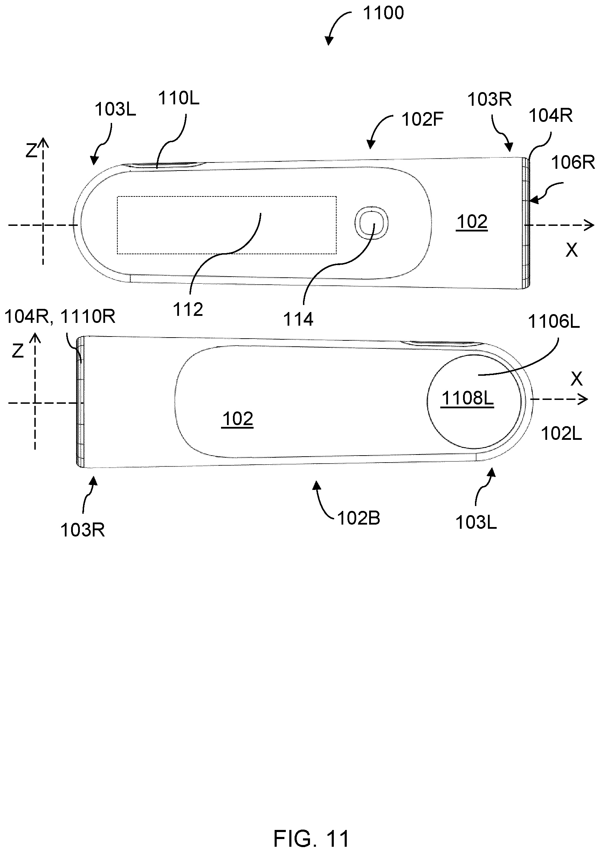

La deuxième électrode 110L peut être positionnée de façon similaire à l'ensemble de capteurs 105 tel que décrit précédemment, c'est-à-dire que les deux électrodes 110R, 110L sont sur la face supérieure 102T (symétrie de positionnement visible sur la face supérieure 102T en figure 2). Plus généralement, les deux électrodes 110L, 110R peuvent être alignées parallèlement à la direction d'extension X. La symétrie de permet de simplifier la prise de mesure. Alternativement, la première électrode ECG 110L peut être sur la face supérieure 102T et la deuxième électrode 110R peut être sur la face avant 102F ou sur la deuxième extrémité 103R, comme représenté sur la figure 11.

La deuxième électrode 110L peut être une électrode ECG et/ou IPG. En touchant simultanément les deux électrodes ECG avec deux doigts de mains différentes, l'utilisateur peut ainsi réaliser un ECG. En variante ou en complément, en touchant simultanément les deux électrodes IPG avec deux doigts de mains différentes, l'utilisateur peut ainsi réaliser une mesure d'impédancemétrie.

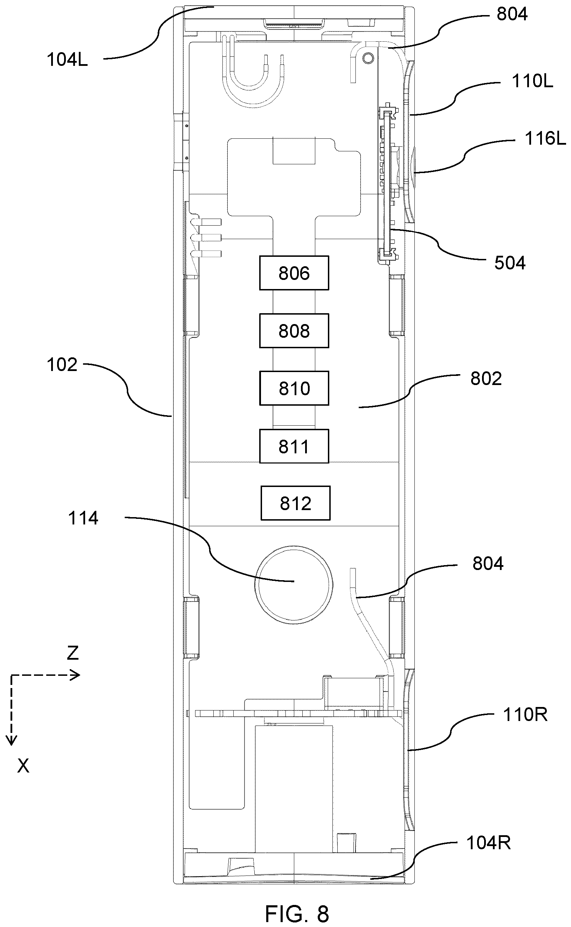

Comme visible sur la figure 8, les deux électrodes 110L, 110R sont connectées au circuit imprimé principal 802, notamment par des contacts électriques 804.

Le dispositif 100 peut comprendre un module ECG 810 connecté au deux électrodes ECG 110L, 110R. Généralement, le module ECG 810 comprend un convertisseur analogique-numérique (ADC) et un processeur. Le module ECG 810 peut être monté sur le circuit imprimé principal 802.

Le module ECG 810 est configuré pour récupérer des signaux électriques venant du corps humain via les électrodes ECG 110L, 110R et pour, après traitement, générer un électrocardiogramme.

Dans un mode de réalisation, le module ECG 810 est configuré pour imposer un potentiel sur l'une des deux électrodes ECG et le potentiel à l'autre électrode est laissé libre par le module ECG 810. De la sorte, le potentiel de cette électrode correspond au potentiel du corps de l'utilisateur (lorsqu'il y a contact) et varie en fonction notamment des battements du coeur de ce dernier.

Le dispositif 100 peut comprendre un module IPG 811 connecté au deux électrodes IPG 110L, 110R. Généralement, le module IPG 811 comprend un convertisseur analogique-numérique (ADC) et un processeur. Le module IPG 811 peut être monté sur le circuit imprimé principal 802.

Le module IPG 811 est configuré pour injecter un courant électrique dans le corps humain via les électrodes IPG 110L, 110R et pour, après traitement, déterminer par exemple une composition corporelle de l'utilisateur.

Dans un mode de réalisation, le dispositif comprend en outre un module d'onde 812 configuré pour calculer une vitesse de propagation d'une onde de pouls dans un bras d'un utilisateur sur la base des signaux reçus du module ECG et du module optique. Généralement, le module d'onde 812 comprend un convertisseur analogique-numérique (ADC) et un processeur. Le module d'onde 812 peut être monté sur le circuit imprimé principal 802 ou sur le circuit imprimé optique 504.

### Les capteurs physiologiques additionnels aux extrémités

Dans un mode de réalisation, le dispositif 100 comprend en outre un capteur physiologique additionnel 106L au niveau de la première extrémité 103L, qu'on nomme premier capteur physiologique additionnel 106L.

Le premier capteur physiologique additionnel 106L comprend une surface fonctionnelle 108L. Par surface fonctionnelle 108L, il est signifié une surface destinée à être positionnée face à l'utilisateur, pour interagir avec celui-ci, avec ou sans contact, pour l'obtention de la mesure physiologique par le premier capteur physiologique 106L.

Par exemple, le premier capteur physiologique additionnel 106L peut être un stéthoscope électronique, avec un capteur piézo-électrique et une membrane d'amplification destinée à être positionnée sur l'utilisateur. Dans ce cas, la surface fonctionnelle 108L comprend la membrane d'amplification. La membrane d'amplification est notamment la partie visible par l'utilisateur du capteur piézoélectrique.

Par exemple, le premier capteur physiologique additionnel 106L peut être un thermomètre, avec un capteur de type thermopile et une lentille. La lentille peut être entourée d'un cône. Dans ce cas, la surface fonctionnelle 108L comprend la lentille et, le cas échéant, le cône. La lentille et le cône sont notamment les parties visibles par l'utilisateur du thermomètre.

Par exemple, le premier capteur physiologique additionnel 106L peut être un spiromètre avec un capteur de volume et/ou de débit d'air et un embout buccal. Dans ce cas, la surface fonctionnelle 108L comprend l'embout buccal. L'embout buccal est notamment la partie visible par l'utilisateur du spiromètre.

Le premier capteur physiologique additionnel 106L est positionné au niveau de la première extrémité 103L et sa surface fonctionnelle 108L est inscrite dans le bord 104L. Cela signifie que, dans une projection selon la direction d'extension X dans un plan transversal YZ au niveau de l'extrémité 103L, la surface fonctionnelle 108L est positionnée à l'intérieur du bord 104L. Formulé autrement, la projection de surface fonctionnelle 108L est comprise dans la projection de la face latérale.

Grâce à ces caractéristiques, la préhension du dispositif 100 n'est pas gênée par le capteur physiologique 106L. En particulier, l'utilisateur peut tenir le dispositif 100 par une extrémité, en ayant la main (par exemple la paume) dans le prolongement de la direction d'extension. Cette caractéristique permet aussi de ranger aisément le dispositif 100, par exemple dans une poche ou dans une boîte.

A cet égard, dans un mode de réalisation, la surface fonctionnelle 108L est positionnée à l'intérieur du volume défini par le boîtier 102 (et donc par le bord 104L au niveau de l'extrémité 103L). Le capteur 106L n'est donc pas en saillie hors du boîtier 102. Toutefois, pour certains capteurs, notamment ceux qui nécessitent un contact, comme le stéthoscope, la surface fonctionnelle 108L (par exemple la membrane) peut s'écarter d'au plus 5 mm hors du volume défini par le boîtier 102 le long de la direction principale X, voire au plus 2 mm.

Dans un mode de réalisation, le dispositif 100 comprend un deuxième capteur physiologique additionnel 106R au niveau de la deuxième extrémité 103R. Ce deuxième capteur physiologique 106R est défini de façon similaire au premier capteur physiologique additionnel 106L.

Dans un mode de réalisation, illustré sur la figure 1, le premier capteur physiologique additionnel 106L est un stéthoscope électronique et le deuxième capteur physiologique additionnel 106R est un capteur de température.

En variante, le premier capteur physiologique additionnel 106L est un capteur de température et le deuxième capteur physiologique additionnel 106R est un stéthoscope électronique.

### Variante du dispositif

Les figures 11 et 12 illustrent un autre mode de réalisation d'un dispositif 1100. Ce mode de réalisation est similaire au dispositif 100, à l'exception d'un capteur physiologique 106L, 106R qui n'est pas inscrit dans un bord comme précédemment décrit.

Sur la figure 11, il s'agit du premier capteur physiologique 1106L qui est modifié, mais ce pourrait être le deuxième capteur physiologique 1106R.

Dans ce mode de réalisation, un capteur physiologique 1106L comprend une face fonctionnelle 1108L qui est disposée sur la face avant 102F ou la face arrière 102R. Dans l'exemple illustré, la surface fonctionnelle 1108L est positionné sur la face arrière 102R, du côté opposé à l'affichage 112 et l'interface mécanique 114. En particulier, ce capteur 1106L est un stéthoscope et l'interface fonctionnelle 1108L est une membrane.

Toutefois, le capteur physiologique 1106L demeure à proximité de l'extrémité 103L, avec notamment « à proximité » qui signifie dans la moitié ou le quart de la longueur L du dispositif 1000.

L'utilisateur peut tenir le boîtier 102 entre l'interface physique 114 et le deuxième bord 104R pour appliquer la membrane 1108L sur le torse.

Comme décrit précédemment, la figure 12 permet d'illustrer un autre mode de réalisation concernant la deuxième électrode 1100R. Ce mode de réalisation est sans lien direct avec le stéthoscope précédemment décrit.

En effet, la deuxième électrode 1100R peut être positionnée sur tout ou partie du bord 104R de l'extrémité 103R. Ainsi, le contact ne s'effectue plus par le doigt mais par la paume de la main, en position de manipulation à deux mains.

Le capteur peut être réalisé par un dépôt métallique sur le bord ou par ou un ajout d'une pièce métallique.

Comme expliqué précédemment, la surface de contact 402 présente ici une forme concave. En d'autres mots, la surface de contact 402 présente une forme bombée vers l'intérieur du boitier 102. Ce mode de réalisation est sans lien direct avec le stéthoscope et la deuxième électrode précédemment décrit.

### Le dispositif et son environnement

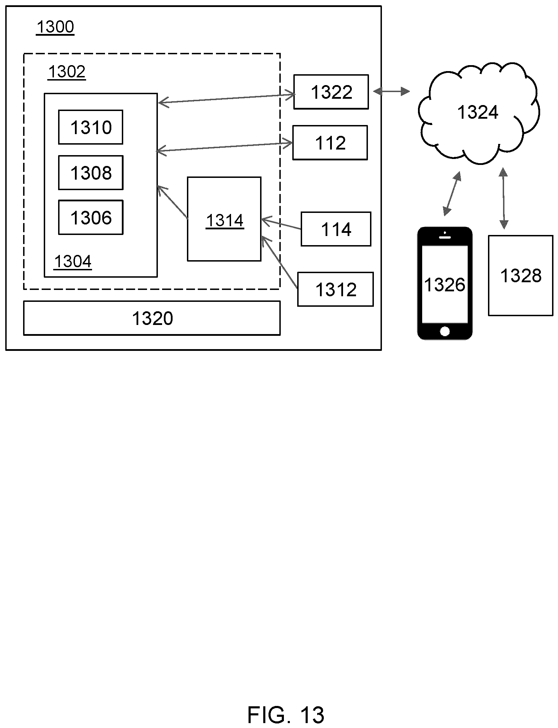

La figure 13 illustre un schéma de l'architecture d'un dispositif 100, 1300 (référencé 1300 sur cette figure) tel que décrit et de son environnement.

Le dispositif 1300 comprend une unité de contrôle 1302 avec une circuiterie de contrôle 1304 comprenant un processeur 1306, une mémoire 1008 et une interface I/O 1310 (« In/Out » en anglais ou « Entrée/Sortie » en français) pour communiquer avec les autres composants.

La mémoire 1308 stocke des programmes, instructions ou autres permettant à la fois la navigation sur le dispositif 1300 ainsi que la prise des mesures (algorithmes notamment). La mémoire 1308 en particulier se décompose en une mémoire volatile, de type RAM, et une mémoire non-volatile, de type flash (ou ROM ou SSD).

L'unité de contrôle 1302 est configurée pour commander le module ECG, le module optique, le module de force, le module de pression et/ou le module d'onde. L'unité de contrôle 1302 est notamment configurée pour commander la mesure simultanée d'un ECG, via le module ECG, et d'une mesure optique, via le module optique.

L'unité de contrôle 1302 est notamment arrangée sur le circuit imprimé principal 802. L'unité de contrôle 1302 peut être constituée de plusieurs sous unités, arrangés sur le circuit imprimé principal 802 et sur le circuit optique 504. Le dispositif 1300 comprend un ou plusieurs capteurs 1312 (tous les capteurs décrits précédemment sont schématisés sous une seule référence 1312).

L'unité de contrôle 1302 comprend typiquement un module d'interface 1314 faisant interface entre les capteurs 1312 et l'interface I/O 1310 de la circuiterie de contrôle 1304. Le module d'interface 1314 comprend notamment des ADC, des filtres, des amplificateurs, etc.

Le dispositif 1300 comprend en outre l'affichage 112, qui communique avec l'interface I/O 1310, et l'interface physique 114 qui communique avec le module d'interface 1314 pour la navigation dans le menu de l'affichage 112.

Pour alimenter les différents composants en énergie électrique, le dispositif 1300 comprend une batterie 1320, par exemple une pile ou une batterie rechargeable. La batterie 1320 est configurée pour alimenter notamment l'unité de contrôle 1302, l'affichage 112 et les capteurs 1312.

Enfin, pour la connectivité, le dispositif 1300 comprend un module de communication sans fil 1322 (Bluetooth, BLE, Wifi, cellulaire, etc.), relié à la circuiterie de contrôle 1302. Le module 1322 permet de communiquer, via un réseau de communication 1324 à un terminal mobile 1326 (par exemple un téléphone portable de type smartphone) et/ou un serveur distance 1328. Les données physiologiques ainsi acquises par le dispositif 1300 peuvent être stockées, analysées, traitées dans le serveur 1328 et affichées par le terminal mobile 1326. Le terminable mobile 1326 peut aussi servir de relai entre le dispositif 1300 et le serveur 1328 (par exemple dans le cas d'une communication Bluetooth ou BLE).

## Revendications

1. Ensemble optique comprenant :
- une unité optique (116L) comprenant un capteur optique (906),
- un circuit imprimé (504) déformable, l'unité optique (116L) étant physiquement montée et électroniquement connectée au circuit imprimé (504), et
- un capteur de force (502) configuré pour déterminer une information relative à la déformation du circuit imprimé (504).

2. Ensemble selon la revendication 1, dans lequel l'unité optique (116L) comprend une surface d'interaction (604) configurée pour être en contact avec un utilisateur.

3. Ensemble selon la revendication 2, dans lequel l'unité optique (116L) comprend un dôme (902) qui définit la surface d'interaction (604), le dôme (902) étant monté sur le circuit imprimé (504) de sorte que le dôme (902) reprenne les efforts du l'utilisateur vers le circuit imprimé (504).

4. Ensemble selon l'une quelconque des revendications précédentes, dans lequel le capteur de force (502) est un capteur de déformation piézoélectrique.

5. Ensemble selon l'une quelconque des revendications précédentes, dans lequel le circuit imprimé (504) comprend une face avant (608) et une face arrière (610), l'unité optique (116L) étant montée sur la face avant (608) et le capteur de force (502) étant monté sur la face arrière (610).

6. Ensemble selon la revendication 5, dans lequel le capteur de force (502) est monté sensiblement en vis-à-vis de l'unité optique (116L).

7. Ensemble selon l'une quelconque des revendications précédentes, comprenant en outre une électrode (110L) comprenant une surface de contact (402) configurée pour être en contact avec un utilisateur, l'unité optique (116L) étant mobile par rapport à la surface de contact (402).

8. Dispositif (100) comprenant un boitier (102) et l'ensemble optique selon l'une quelconque des revendications précédentes, l'unité optique (116L) étant mobile par rapport au boitier (102) entre une position de repos et une position déplacée.

9. Dispositif (100) selon la revendication 8, dans lequel l'ensemble est selon la revendication 2, et dans lequel au moins la surface d'interaction (604) de l'unité optique (116L) fait saillie hors du boitier (102) dans la position de repos.

10. Dispositif (100) selon la revendication 8 ou 9, dans lequel le circuit imprimé déformable (504) est fixé à chacune de ses extrémités au boitier (102), par exemple par une pince (506).

11. Dispositif (100) selon l'une quelconque des revendications 8 à 10, dans lequel le boitier (102) présente une forme allongée selon une direction d'extension (X), dans lequel le circuit imprimé (504) est fixé au boîtier (102), au niveau des deux extrémités opposées (504a, 504b) le long de la direction d'extension (X).

12. Dispositif (100) selon l'une quelconque des revendications 8 à 11, dans lequel le dispositif (100) est configuré pour fournir à un utilisateur une information représentative de la force exercée sur l'unité optique (116L) dans la position déplacée.

13. Dispositif (100) selon l'une quelconque des revendications 8 à 12, dans lequel le dispositif comprend un écran (112), l'écran (112) affiche un intervalle recommandé de force exercée sur l'unité optique (116L).

14. Dispositif (100) selon l'une quelconque des revendications 8 à 13, dans lequel le capteur optique (906) comprend une source de lumière (908) et un récepteur de lumière (910), le dispositif (100) comprenant un module optique (612) configuré pour générer les instructions permettant à la source de lumière (908) d'émettre de la lumière et configuré pour recevoir des signaux du récepteur de lumière (910), notamment pour déterminer un rythme cardiaque ou une saturation en oxygène du sang de l'utilisateur.

15. Dispositif (100) selon l'une quelconque des revendications 8 à 14, dans lequel le dispositif (100) comprend un module de pression (808) configuré pour calculer une pression artérielle de l'utilisateur sur la base des signaux reçus par le module optique (612) et le capteur de force (502).
